Europäisches Patentamt

European Patent Office

Office européen des brevets

(19)

(11) Veröffentlichungsnummer: **0 211 351**
B1

## (12) EUROPÄISCHE PATENTSCHRIFT

(45) Veröffentlichungstag der Patentschrift:
24.10.90

(51) Int. Cl.⁵: **C09C 1/00**

(21) Anmeldenummer: 86110282.0

(22) Anmeldetag: 25.07.86

(54) Eisenoxidbeschichtete Perlglanzpigmente.

(30) Priorität: 07.08.85 DE 3528256

(43) Veröffentlichungstag der Anmeldung:
25.02.87 Patentblatt 87/9

(45) Bekanntmachung des Hinweises auf die Patenterteilung:
24.10.90 Patentblatt 90/43

(84) Benannte Vertragsstaaten:
BE CH DE FR GB IT LI NL

(56) Entgegenhaltungen:
DE-B- 1 467 468
FR-A- 2 352 867
US-A- 4 086 100
US-A- 4 192 691

**Die Akte enthält technische Angaben, die nach dem
Eingang der Anmeldung eingereicht wurden und die
nicht in dieser Patentschrift enthalten sind.**

(73) Patentinhaber: MERCK PATENT GESELLSCHAFT MIT
BESCHRÄNKTER HAFTUNG, Frankfurter
Strasse 250 Postfach 4119, D-6100 Darmstadt(DE)

(72) Erfinder: Franz, Klaus-Dieter, Dr., Insterburger
Strasse 12, D-6233 Kelkheim(DE)
Erfinder: Ambrosius, Klaus, Dr., Waldschmidtstrasse 65,
D-6000 Frankfurt am Main(DE)

## Beschreibung

Die Erfindung betrifft Perlglanzpigmente auf Basis von mit Metalloxiden überzogenen plättchenförmigen Substraten, insbesondere Glimmer, wobei die Metalloxidschicht sowohl Titan als auch Eisen enthält.

Eisenhaltige Glimmerschuppenpigmente sind bereits mehrfach beschrieben worden und werden auch seit vielen Jahren mit Erfolg angewendet. Dabei werden sowohl Pigmente beschrieben, bei denen Eisenoxid zusammen mit einem anderen Metalloxid, insbesondere Titandioxid, auf die Glimmerplättchen aufgefällt wird, als auch Pigmente, bei denen die Fällungen nacheinander erfolgen.

Im US-Patent 3 087 828 wird beschrieben, daß durch Abscheiden einer $Fe_2O_3$-Schicht auf eine $TiO_2$-Schicht goldfarbene Pigmente erhalten werden, die beim Glühen einen rötlichen Farbton annehmen. In der deutschen Patentschrift 19 59 998 sind Pigmente beschrieben, die auf Glimmer zunächst eine Mischschicht von Titan- und Eisenoxid und darauf eine Deckschicht aus Titan und/oder Zirkondioxid besitzen.

In der deutschen Patentschrift 22 44 298 wird ein Verfahren zur Herstellung von goldfarbenen Perlglanzpigmenten beschrieben, bei dem ein mit $TiO_2$ und/oder $ZrO_2$ beschichtetes Glimmerpigment zunächst mit Eisen(II)-hydroxid beschichtet wird, das dann zu $Fe_2O_3$ oxidiert wird.

In der deutschen Offenlegungsschrift 23 13 331 sind vorteilhafte eisenhaltige Pigmente beschrieben, bei denen das Eisenoxid in bestimmten definierten Kristallmodifikationen vorliegt.

Schließlich werden in der deutschen Offenlegungsschrift 27 23 871 Glimmerpigmente beschrieben, die auf einer sehr dünnen $TiO_2$ oder $Al_2O_3$-Schicht eine dicke $Fe_2O_3$-Schicht tragen.

In all den beschriebenen Fällen wird das $TiO_2$ als Anatas aufgebracht. Dabei ist festzustellen, daß beim Glühen des Pigments das gefällte Eisenoxid sehr stark in die $TiO_2$-Schicht eindiffundiert, so daß selbst in den Fällen, in denen zunächst getrennte Schichten von $TiO_2$ und $Fe_2O_3$ aufgefällt wurden, nach dem Glühen eine Mischschicht, bestehend im wesentlichen aus Pseudobrookit, vorliegt.

In der deutschen Patentschrift 25 22 572 wurde auch schon vorgeschlagen, mit $TiO_2$ in der Rutilform beschichtete Glimmerpigmente mit einer zusätzlichen Deckschicht aus färbenden Metalloxiden zu versehen, wobei auch $Fe_2O_3$ genannt ist. Tatsächlich wurden auch solche Beschichtungen mit relativ geringen Eisenoxidmengen vorgenommen. Dabei zeigt sich der gleiche Effekt, daß beim Glühen durch Diffusion des Eisenoxids auf der Basisrutilschicht eine Mischschicht aus Pseudobrookit entsteht.

Es wurde nun jedoch überraschenderweise festgestellt, daß das Verhalten von Rutil und Anatas in bezug auf die Diffusion von Eisenoxid beim Glühen deutlich unterschiedlich ist. Während im Falle von Anatas auch große Mengen von Eisenoxid beim Glühen praktisch vollständig in die $TiO_2$-Schicht eindiffundieren wurde festgestellt, daß im Falle einer Rutilschicht die Diffusion so gering ist, daß sich auf der Rutilschicht nur eine relativ dünne Pseudobrookitschicht bildet, während die Restmenge der Eisenoxidfällung tatsächlich in Form von $Fe_2O_3$ vorliegt.

Tatsächlich lassen sich bei den in bezug auf Glanz und Stabilität deutlich verbesserten neuen Pigmenten im Gegensatz zu den bekannten Pigmenten, bei denen die Eisenoxidschicht auf eine Anatasschicht aufgefällt ist, durch Röntgenbeugung diskrete Phasen von Rutil-$TiO_2$ und von $Fe_2O_3$ nebeneinander nachweisen.

Überraschenderweise besitzen Pigmente mit einem solchen 3-Schichtenaufbau sowohl deutlich verbesserte coloristische Eigenschaften (verbesserte Farbstärke und Brillanz und verbessertes Deckvermögen) als auch eine verbesserte chemische Beständigkeit (beispielsweise in bezug auf die Photoaktivität oder in Schmelzflüssen für Glasuren und Email).

Gegenstand der Erfindung sind daher Perlglanzpigmente auf Basis von mit Metalloxiden überzogenen plättchenförmigen Substraten, insbesondere Glimmer, wobei die Metalloxidschicht sowohl Titan als auch Eisen enthält, die dadurch gekennzeichnet sind, daß das Pigment einen Mehrschichtenaufbau besitzt, wobei auf eine erste Schicht von $TiO_2$ in der Rutilform eine Schicht von Pseudobrookit und eine Eisenoxidschicht folgt.

Gegenstand der Erfindung ist auch ein Verfahren zur Herstellung von Perlglanzpigmenten durch Beschichten von plättchenförmigen Substraten, insbesondere Glimmer mit einer Eisen und Titan enthaltenden Metalloxidschicht, das dadurch gekennzeichnet ist, daß zunächst eine Titandioxid- bzw. Titandioxidaquatschicht aufgefällt wird, wobei diese Fällung in an sich bekannter Weise so vorgenommen wird, daß beim Glühen eine Rutilschicht entsteht, und daß vor oder nach dem Glühen dieses Pigments eine Eisenoxid- bzw. Hydroxidschicht aufgefällt wird und das Pigment abgetrennt, ggf. gewaschen und geglüht wird.

Entscheidend im Zusammenhang mit der vorliegenden Erfindung ist es, daß die $TiO_2$-Schicht so aufgebracht wird, daß beim Glühen die Rutilmodifikation entsteht. Dies kann nach einem der bekannten Verfahren geschehen, wobei Fremdionen, insbesondere Zinn(IV), in die Schicht eingebaut wird. Diese Verfahren sind z. B. in den deutschen Patentschriften 22 14 545 und 25 22 572 beschrieben, wobei durch Einbau von Zinndioxid in Glimmernähe oder in diskreten Schichten zwischen dem $TiO_2$ die Rutilisierung erzwungen wird. Es sind aber auch andere Verfahren bekannt, wie z. B. der Einbau von Zinkoxid entsprechend der CS-PS 208,578 oder der Einbau von Eisen(III) in der $TiO_2$-Schicht entsprechend der DE-PS 19 59 998, die zu im Rahmen der vorliegenden Erfindung nutzbaren Rutilschichten führen.

Für die Auffällung der $TiO_2$-Schicht sind im wesentlichen 2 Verfahren bekannt. So kann die Fällung, wie z. B. im US-Patent 30 87 828 beschrieben, durch Zugabe einer schwefelsauren Titanylsulfatlösung zur Glimmersuspension und deren Hydrolyse durch Erhitzen auf etwa 100 °C erfolgen, wobei die Schichtdicke und die damit verbundene Interferenzfarbe von vornherein durch die Menge des anwesenden Titanylsulfats vorgegeben ist.

Die Fällung kann aber auch so wie beispielsweise in der deutschen Patentschrift 20 09 566 beschrieben durchgeführt werden. Dabei wird zu einer auf etwa 50-100 °C, insbesondere 70-80 °C erhitzten Glimmersuspension langsam eine wäßrige Titansalzlösung zugegeben, und es wird durch gleichzeitiges Zudosieren einer Base, wie z. B. wäßrige Ammoniaklösung oder eine wäßrige Alkalilauge, ein weitgehend konstanter pH-Wert von etwa 0,5-5, insbesondere etwa 1,5-2,5 eingehalten. Sobald die gewünschte Schichtdicke der $TiO_2$-Fällung erreicht ist, wird die Zugabe der Titansalzlösung gestoppt.

Um die Titandioxidschicht in der gewünschten Rutilmodifikation zu erhalten, wird die Fällung auf bekannte Weise so modifiziert, daß entweder in der Glimmersuspension ein Zinnsalz anwesend ist, das gemeinsam mit dem Titanylsulfat hydrolysiert wird, oder daß nach Auffällung einer dünnen $TiO_2$-Schicht eine $SnO_2$-Zwischenschicht und danach wieder eine $TiO_2$-Schicht aufgefällt wird, wobei diese alternierenden Fällungen ggf. mehrmals wiederholt werden können.

Auch die Eisenoxidschicht kann nach bekannten Verfahren aufgefällt werden. Dabei kann sowohl von Eisen(III)-salzen ausgegangen werden, wie es z. B. in der deutschen Patentschrift 14 67 468 beschrieben ist, als auch von Eisen(II)-salzen, wie in der deutschen Patentschrift 22 44 298 beschrieben, wobei der zunächst gebildete Überzug von Eisen(II)-hydroxid zum Eisen(III)-oxidhydrat oxidiert wird.

Die Auffällung der Eisenoxidschicht kann sowohl auf eine geglühte und damit schon rutilisierte $TiO_2$-Schicht erfolgen als auch direkt nach der $TiO_2$-Fällung auf das ungeglühte Pigment. Auch im letzteren Fall tritt über raschenderweise beim anschließenden Glühen nur eine sehr geringe Diffusion des Eisens in die $TiO_2$-Schicht ein, obwohl beim Glühen relativ hohe Temperaturen von etwa 700 bis etwa 950°C, insbesondere von etwa 800 bis etwa 900°C angewendet werden.

Da jedoch auch bei der erfindungsgemäßen Unterlage einer Rutil-$TiO_2$-Schicht eine geringe Diffusion von Eisenoxid in die $TiO_2$-Schicht unter Bildung von Pseudobrookit nicht ganz vermieden werden kann, sollte die $TiO_2$-Schicht eine gewisse Mindeststärke von etwa 40 nm besitzen, damit ein 3-Schichtenaufbau $TiO_2$/Pseudobrookit/$Fe_2O_3$ zustande kommt. Schichtdicken der $TiO_2$-Schicht vor dem Glühen von etwa 40 bis etwa 200 nm und insbesondere Dicken von etwa 40 bis etwa 150 nm sind bevorzugt.

Wesentlich für den erfindungsgemäßen 3-Schichtenaufbau ist jedoch insbesondere auch die Schichtdicke der aufgefällten $Fe_2O_3$-Schicht. Diese sollte in jedem Fall so groß sein, daß nach dem Glühen und der dabei stattfindenden Bildung einer Zwischenschicht von Pseudobrookit noch eine reine $Fe_2O_3$-Schicht an der Oberfläche der Pigmentteilchen verbleibt. Aufgefällt wird daher eine $Fe_2O_3$-Schicht von mindestens etwa 15 nm und bevorzugt Schichten mit etwa 15 bis etwa 50 nm, insbesondere solche von etwa 20 bis etwa 40 nm.

Sowohl in die $TiO_2$- als auch in die $Fe_2O_3$-Schicht können noch Dotierstoffe, insbesondere weitere gefärbte oder farblose Metalloxide eingebaut werden. Als solche kommen beispielsweise in Frage Verbindungen von Aluminium(III), Silizium(IV), Zirkonium(IV), Chrom(III), Bor(III) und Phosphor(V). Diese Dotierstoffe werden ggf. jeweils in Mengen von etwa 0 bis 2 Gew. % eingebracht. Insgesamt sollte jedoch eine Menge von etwa 2 bis etwa 5 Gew.% nicht überschritten werden. Falls Dotierstoffe in eine der Schichten oder auch alle Schichten eingebaut werden sollen, können diese der Glimmersuspension, einer der zugegebenen Salzlösungen oder ggf. auch der zudosierten Base in Form von wasserlöslichen Salzen beigegeben werden. Die Dotierstoffe sind in der Regel in der oder den Metalloxidschichten homogen verteilt. Es ist aber auch möglich und ggf. vorteilhaft, eine Anreicherung entweder in Glimmernähe oder an der Oberfläche des Pigments vorzunehmen.

Es ist weiterhin möglich, die Pigmente einer Nachbeschichtung oder Nachbehandlung zu unterziehen, die die Licht-, Wetter- und chemische Stabilität weiter erhöht, oder die Handhabung des Pigments, insbesondere die Einarbeitung in unterschiedliche Medien, erleichtert. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Aufgrund der bereits ohne diese zusätzlichen Maßnahmen sehr guten Eigenschaften der erfindungsgemäßen Pigmente machen diese ggf. noch aufgebrachten Stoffe nur etwa 0 bis 5, insbesondere etwa 0 bis 3 Gew.% des gesamten Pigments aus.

Die erfindungsgemäßen Pigmente können wie die bisher bekannten Pigmente verwendet werden, also z. B. zur Pigmentierung von Kunststoffen, Farben, Lacken, Körperpflegemitteln und Kosmetika, wegen der guten chemischen Beständigkeit aber auch in Schmelzflüssen für Glasuren und Email.

Beispiel 1

Entsprechend Beispiel 1 A der DE-PS 22 14 545 werden 100 g Glimmer nacheinander mit 0,8 g $SnO_2$ und 30 g $TiO_2$ in wäßriger Suspension beschichtet. Das suspendierte ungeglühte Pigment zeigt dabei eine schwach gelbe Interferenzfarbe. Nach Abstellen des Rührwerks und Absitzen des Pigments wird die überstehende Flüssigkeit abgezogen, und es werden 2500 ml Wasser sowie 81 g wasserfreies $FeCl_3$ und 16 g Natriumacetat zugegeben. Nach 1stündigem Erhitzen auf 70-80 °C wird abfiltriert, chloridfrei gewaschen, getrocknet und 30 min bei 850 °C calciniert. Man erhält ein Pigment mit tiefdunkler, goldener Körper- und Interferenzfarbe, dessen Röntgenbeugungswerte (Debye-Scherrer-Diagramm) erkennen lassen, daß auf dem Muscovit diskrete Schichten von Rutil, Pseudobrookit und Haematit vorliegen.

Das Pigment besitzt eine gute Beständigkeit in Glasuren und zeigt im Kronos-Test keine Photoaktivität.

Vergleichsbeispiel 1 A

Es wird analog Beispiel 1 gearbeitet, jedoch ohne die SnO$_2$-Fällung. Man erhält ein Pigment mit hellgelber Körper- und gelber Intereferenzfarbe, dessen Beschichtung aufgrund der Röntgenbeugungswerte aus Anatas und Pseudobrookit besteht. Die Beständigkeit des Pigments in Glasuren ist schlecht, und im Kronos-Test zeigt es eine Photoaktivität.

Beispiel 2

Entsprechend Beispiel 2 der DE-PS 25 22 572 werden durch alternierende Beschichtung von Glimmer mit TiO$_2$, SnO$_2$ und TiO$_2$ 109 g eines Pigments mit blauer Interferenzfarbe hergestellt. Nach Absitzen des Pigments wird dekantiert und mit 2500 ml Wasser aufgefüllt. Zu der auf 75 °C erhitzten und mit Ammoniak auf pH 6-7 eingestellten Suspension wird unter gleichzeitigem Einblasen von Luft eine Lösung von 120 g FeSO$_4$. 7 H$_2$O in 400 ml Wasser und 1,5 ml konz. Schwefelsäure langsam zugegeben, wobei durch gleichzeitige Zugabe von Ammoniak der pH-Wert weitgehend konstant gehalten wird. Danach wird das Pigment abgetrennt, sulfatfrei gewaschen, getrocknet und 30 min bei 850 °C geglüht. Man erhält ein goldenes, glänzendes Pigment mit grüner Interferenzfarbe und hohem Deckvermögen, das aufgrund der Röntgenbeugungswerte Schichten von Rutil, Pseudobrookit und Haematit besitzt. Es zeigt eine gute Stabilität in Glasuren und im Kronos-Test keine Photoaktivität.

Vergleichsbeispiel 2 A

Es wird analog Beispiel 2 gearbeitet, wobei jedoch die SnO$_2$-Zwischenschicht ausgelassen wird. Man erhält ein Pigment mit hellgelber Körper- und grüner Interferenzfarbe, das nach den Röntgenbeugungswerten eine Beschichtung aus Anatas und Pseudobrookit trägt. Es zeigt eine schlechte Stabilität in Glasuren und eine Photoaktivität im Kronos-Test.

Beispiel 3

Entsprechend dem Verfahren nach Beispiel 1 der DE-PS 19 59 998 wird auf 100 g Glimmer in wässeriger Suspension eine Mischfällung aus hydratisiertem Titandioxid und hydratisiertem Eisen(III)oxid aufgebracht. Sobald eine orangefarbene Interferenz erreicht ist, läßt man das Pigment absitzen, dekantiert die überstehende Lösung und füllt mit Wasser auf 2500 ml auf.

Anschließend wird wie in Beispiel 2 beschrieben unter Verwendung von 120 g FeSO$_4$. 7 H$_2$O Eisenoxid aufgefällt. Nach Aufarbeiten und Glühen wie in Beispiel 2 erhält man ein kupferfarbenes Pigment mit blauer Interferenzfarbe und hoher Stabilität, das im Röntgenbeugungsdiagramm Rutil, Pseudobrookit und Haematit zeigt. Es besitzt eine gute Stabilität in Glasuren und keine Photoaktivität im Kronos-Test.

**Patentansprüche**

1. Perlglanzpigment auf Basis von mit Metalloxiden überzogenen plättchenförmigen Substraten, insbesondere Glimmer, wobei die Metalloxidschicht sowohl Titan als auch Eisen enthält, dadurch gekennzeichnet, daß das Pigment einen Mehrschichtenaufbau besitzt, wobei auf eine erste Schicht von TiO$_2$ in der Rutilform eine Schicht von Pseudobrookit und eine Eisenoxidschicht folgt.

2. Perlglanzpigment nach Anspruch 1, dadurch gekennzeichnet, daß der Eisengehalt berechnet als Fe$_2$O$_3$ und bezogen auf das Gesamtpigmentgewicht etwa 10 bis etwa 50 Gew.% beträgt.

3. Perlglanzpigment nach Anspruch 1 oder 2, dadurch gekennzeichnet, daß der Titangehalt berechnet als TiO$_2$ und bezogen auf das Gesamtpigment etwa 20 bis etwa 50 Gew.% beträgt.

4. Verfahren zur Herstellung von Perlglanzpigmenten durch Beschichten von plättchenförmigen Substraten, insbesondere Glimmer, mit einer Eisen und Titan enthaltenden Metalloxidschicht, dadurch gekennzeichnet, daß zunächst eine Titandioxid- bzw. Titandioxidaquatschicht aufgefällt wird, wobei diese Fällung in an sich bekannter Weise so vorgenommen wird, daß beim Glühen eine Rutilschicht entsteht, und daß vor oder nach dem Glühen dieses Pigments eine Eisenoxid- bzw. Hydroxidschicht aufgefällt wird und das Pigment abgetrennt, ggf. gewaschen und geglüht wird.

5. Verfahren nach Anspruch 4, dadurch gekennzeichnet, daß die Titandioxidfällung so vorgenommen wird, daß sich vor dem Glühen eine Schichtdicke der TiO$_2$-Schicht von mindestens 40 nm ergibt.

6. Verfahren nach Anspruch 4 oder 5, dadurch gekennzeichnet, daß die Eisenoxidfällung so vorgenommen wird, daß sich vor dem Glühen eine Schichtdicke von mindestens 20 nm ergibt.

**Claims**

1. Perlescent pigment based on platelet-like substrates, in particular mica, coated with metal oxides, the metal oxide layer containing not only titanium but also iron, characterized in that the pigment has a multilayer structure in which a first layer of TiO$_2$ in the rutile form is followed by a layer of pseudobrookite and an iron oxide layer.

2. Perlescent pigment according to Claim 1, characterized in that the iron content calculated as Fe$_2$O$_3$ and relative to the total pigment weight is about 10 to 50% by weight.

3. Perlescent pigment according to Claim 1 or 2, characterized in that the titanium content calculated as TiO$_2$ and relative to the total pigment is about 20 to about 50% by weight.

4. Process for preparing perlescent pigments by coating platelet-like substrates, in particular mica, with an iron- and titanium-containing metal oxide layer, characterized in that first a titanium dioxide or titanium dioxide hydrate layer is precipitated on, this precipitation being effected in a manner known per se in such a way that a rutile layer is formed on calcination, and in that before or after calcination of this pigment an iron oxide or hydroxide layer is precipitated on, and the pigment is separated off, where appropriate washed and calcined.

5. Process according to Claim 4, characterized in that the titanium dioxide precipitation is carried out in such a way that before calcination a layer thickness of the $TiO_2$ layer of at least 40 nm results.

6. Process according to Claim 4 or 5, characterized in that the iron oxide precipitation is carried out in such a way that before calcination a layer thickness of at least 20 nm results.

## Revendications

1. Pigment nacré à base de substrats en paillettes revêtues d'oxydes métalliques, notamment du mica, la couche d'oxyde métallique comprenant aussi bien du titane que du fer, caractérisé en ce que le pigment possède une structure multicouche, dans laquelle sur une première couche de $TiO_2$ sous forme rutile se succèdent une couche de pseudobrookite et une couche d'oxyde de fer.

2. Pigment nacré selon la revendication 1, caractérisé en ce que la teneur en fer calculée en $Fe_2O_3$ et rapportée au poids total de pigment représente d'environ 10 à environ 50% en poids.

3. Pigment nacré selon la revendication 1 ou 2, caractérisé en ce que la teneur en titane, calculée en $TiO_2$ et rapportée au poids total de pigment représente d'environ 20 à environ 50% en poids.

4. Procédé de préparation de pigment nacré par revêtement de substrats en paillettes, notamment du mica, avec une couche d'oxyde métallique comprenant du fer et du titane, caractérisé en ce qu'on précipite d'abord une couche de dioxyde de titane ou de dioxyde hydraté de titane, la précipitation étant réalisée de manière connue en soi, de sorte qu'à la calcination il se forme une couche de rutile et que, avant ou après la calcination de ce pigment on précipite une couche d'oxyde de fer ou d'hydroxyde de fer et qu'on sépare le pigment, le cas échéant on le lave, et on calcine.

5. Procédé selon la revendication 4, caractérisé en ce qu'on réalise la précipitation de dioxyde de titane de telle manière qu'on ait avant calcination une épaisseur de couche de $TiO_2$ d'au moins 40 nm.

6. Procédé selon la revendication 4 ou 5, caractérisé en ce qu'on réalise la précipitation d'oxyde de fer de telle manière qu'on ait avant calcination une épaisseur de couche d'au moins 20 nm.